# EUROPEAN PATENT SPECIFICATION

(11) **EP 3 235 523 B1**
(45) Date of publication and mention of the grant of the patent: **19.09.2018**
(21) Application number: 17000475.8
(22) Date of filing: 22.03.2017
(51) Int. Cl.: A61L 27/20, A61L 27/36, A61F 2/04

(54) **TRACHEAL PROSTHESIS AND METHOD OF ITS CONSTRUCTION**
TRACHEAL PROTHESE UND EIN VERFAHREN ZU DEREN HERSTELLUNG
PROTHÉSE TRACHÉALE ET UN PROCÉDÉ POUR SA PRÉPARATION

(30) Priority: 19.04.2016 PL 41689916
(43) Date of publication of application: 25.10.2017
(73) Proprietor: Politechnika Lódzka, 90-924 Lódz (PL)
(72) Inventor: Kolodziejczyk, Marek, 92-511 Lódz (PL); Pankiewicz, Teresa, 93-347 Lódz (PL); Rytczak, Przemyslaw, 94-040 Lódz (PL); Ludwicka, Karolina, 93-165 Lódz (PL); Bielecki, Stanislaw, 92-117 Lódz (PL)
(74) Representative: Kaczur-Kaczynska, Ewa

(56) References cited:
- EP-A1- 0 464 755
- EP-A2- 2 371 401

## Description

The subject-matter of the invention refers to a tracheal prosthesis and method of its construction. The prosthesis - according to the invention - made from BioNanoCellulose, is intended to reconstruct a natural part of a human trachea.

The patent description no. PL 216702 provides information on biomaterial having the characteristics of cartilage and the method of its production, intended for implants applied in reconstruction-restoration surgery, to restore, inter alia, a trachea, from microbial cellulose developed under the stationary culture of *Gluconacetobacter xylinus* bacteria at the production substrate containing glucose, yeast extract, peptone, MgSO₄ x 7H₂O, Na₂HPO₄, citric acid, ethanol and distilled water and purified by means of rinsing in hot tap water, boiling in 1% aqueous solution of sodium hydroxide or treating with 1-2% aqueous solution of sodium hydroxide at room temperatures, rinsing in tap water, treating with 1% aqueous solution of acetic acid and re-rinsing with tap water at first and then with distilled water, in which cellulose is produced under the cultivation of bacteria in a flat bio-reactor or in polyethylene tubes, then the produced and purified cellulose is shaped into a spatial construction with its desired shape and subjected to modification based on treating it with 30% aqueous solution of sodium hydroxide for up to 24 hours at room temperatures, rinsing with distilled water, then treating with 10% aqueous solution of acetic acid for at least 2 hours and re-rinsing with distilled water until obtaining cellulosic material with its pH at 5.6-6.8.

The patent description no. PL 220652 provides information on a durable composite of bacterial BioNanoCellulose with perforated polymeric or metal material, intended for the reconstruction of soft and hard tissues, consisting in the placement of sterile perforated polymeric or metallic material in the medium of the production culture of a strain of *Gluconacetobacter xylinus* bacteria comprising 20 weight parts of glucose, 5 weight parts of yeast extract, 5 weight parts of peptone, 2.5 weight parts of MgSO₄ x 7H₂O, 2.7 weight parts of Na₂HPO₄, 1.15 weight parts of citric acid, 10 weight parts of ethanol, up to 1000 weight parts of distilled water, inoculated with an inoculum of the bacteria grown at the inoculated substrate having the same composition and keeping the stationary production culture of the bacteria at a temperature of 27-33 °C until the combination of polymeric or metal material with BioNanoCellulose membrane over its entire surface. The cultivation of the bacteria is run in a bioreactor with its walls inclined at an angle of 90-30° in relation to its base. The perforated polymer or metal material intended to be combined with BioNanoCellulose membrane is suspended in the bioreactor vertically so that its lower edge is flush with the medium surface until the formation of first layers of BioNanoCellulose, and in the subsequent days of its cultivation, the material is gradually lowered into the medium to grow it through with BioNanoCellulose over its entire surface; then the material overgrown with BioNanoCellulose membrane is taken out of the bioreactor, cut out of excess membrane, pressed to its desired thickness, packaged and sterilised.

Some attempts to produce a tracheal prosthesis with the application of tissue material were published in the Biomaterials Journal (2009, 30, 4117-4126).

The patent description PL148980 provides information on a bronchial-tree prosthesis made of a non-reactive elastic (plastic) tube reinforced with a stainless steel wire embedded with the material in the shape of helical coils.

Then, the patent application description EP 1245201 provides information on an artificial trachea comprising a tube consisting of two layers of welded polypropylene with in-between amorphous fibres of coiled collagen obtained via electro-spinning.

The Otolaryngologia Polska [Otolaryngology Poland] Journal (2009, 63(3), pp. 303-305) published an article on attempts under experimental conditions to reconstruct a trachea by means carbon material.

A tracheal prosthesis made from BioNanoCellulose obtained under the production culture of *Gluconacetobacter xylinus* bacteria, in the form of a tube-shaped segment with its inner diameter equal to the diameter of the human trachea interior - **according to the invention** - is characterised in that its walls have rings made of mercerised BioNanoCellulose obtained under the bacterial culture of *Gluconacetobacter xylinus,* having the characteristics of cartilage, with its height of 7-10 mm, arranged at a distance of 4-7 mm from one another, forming the prosthesis frame, which is overgrown with native BioNanoCellulose membrane obtained under the bacterial culture of *Gluconacetobacter xylinus.*

The method of construction of this tracheal prosthesis specified above, from BioNanoCellulose under the stationary production culture of a strain of *Gluconacetobacter xylinus* bacteria at the production substrate containing 20 weight parts of glucose, 5 weight parts of yeast extract, 5 weight parts of peptone, 2.5 weight parts of MgSO₄ x 7H₂O, 2.7 weight parts of Na₂HPO₄, 1.15 weight parts of citric acid, 10 weight parts of ethanol, up to 1000 weight parts of distilled water, inoculated with an inoculum of the bacteria kept at the inoculated substrate having the same composition, at a temperature of 30°C within 3-28 days and then mercerised in an aqueous solution of sodium hydroxide, under the process of overgrowth of the material with native BioNanoCellulose membrane suspended vertically in the bioreactor with walls inclined in relation to the base, having the production culture substrate of a strain of *Gluconacetobacter xylinus* bacteria at its raised composition and lowered gradually into the substrate on subsequent days of cultivation of the bacteria under the conditions specified above, in the course of growth of native BioNanoCellulose layers at the medium surface until overgrowth of the suspended material with BioNanoCellulose membrane over its entire surface, **according to the invention** is characterised by the following: at first under this bacterial culture a construct is made from native BioNanoCellulose in the shape of a cylinder with its outer diameter of 3-8 cm together with a concentric, throughway hole having its diameter equal to the diameter of the human trachea interior and then this construct is subjected to the process of mercerisation and then - using this prosthesis frame made in this manner from mercerised BioNanoCellulose, having the characteristics of cartilage - rings with their height of 7-10 mm are cut out, and then they are - connected or not connected, arranged one above the other at a distance from each other equal to 4-7 mm, after their sterilisation - subjected to the process of overgrowing with native BioNanoCellulose through lowering subsequent rings into the medium placed in the bioreactor with its walls inclined in relation to its base, in the subsequent days of the cultivation, and then - when overgrown with native BioNanoCellulose - these rings are taken out from the bioreactor, a concentric throughway hole with its diameter smaller by 2-3 mm from the diameter of the rings grown into BioNanoCellulose and the prosthesis made in the manner is treated with the process of purification. Such a construct from native BioNanoCellulose in the shape of a cylinder is obtained under the production culture of bacteria in a glass flask with its side walls inclined in relation to its bottom at an angle of 10-80°, with the diameter of its neck equal to 3-8 cm and the height of the medium layer from the flask bottom to the place where BioNanoCellulose membrane is formed equal to 8-30 cm. This construct made from native BioNanoCellulose is treated with the process of mercerisation upon placing a glass tube with the same diameter as the one of the trachea interior into a hole drilled in it while the process of mercerisation is run using 20-30% aqueous solution of sodium hydroxide for 24 hours at room temperatures. Such rings cut from the construct made from native BioNanoCellulose i.e. the prosthesis frame, are treated - prior to being subjected to their overgrowth with native BioNanoCellulose - with thermal sterilisation at a temperature of 121°C for 20 minutes under pressure of 1013 hPa. These rings when cut out of the prosthesis frame are subjected to overgrowth with native BioNanoCellulose in the bioreactor with its walls inclined at an angle of 10-80° in relation to the base, while - prior to starting the culture - they are lowered into the reactor until the lowest ring gets in contact with the medium surface. The process of purification of the resulting prosthesis consists first in rinsing it with hot tap water, treating with 1% aqueous solution of sodium hydroxide at a temperature of 100°C for 1 hour or 1-2% aqueous solution of sodium hydroxide for 20-24 hours at room temperatures, rinsing in tap water, treating with 1% aqueous solution of acetic acid for 20-24 hours, re-rinsing in tap water, rinsing in distilled water and finally removing excess water off the composite in order to obtain its wall 2-3 mm thick. Then the prosthesis is sterilised and packaged.

According to the invention, this prosthesis is a composite made from BioNanoCellulose transformed into material having the characteristics of cartilage, showing no roughness and native BioNanoCellulose permanently attached to it, which is a qualitatively-new and innovative bio-compatible product in the shape of a tube having a diameter of the human trachea, intended to replace part of the natural trachea affected as a result of injury or disease. The need to fill cavities within the trachea or its partial / complete prosthetics is often due to inborn defects, mechanical injuries suffered by the organ or diseases most frequently inoperable tumoral ones.

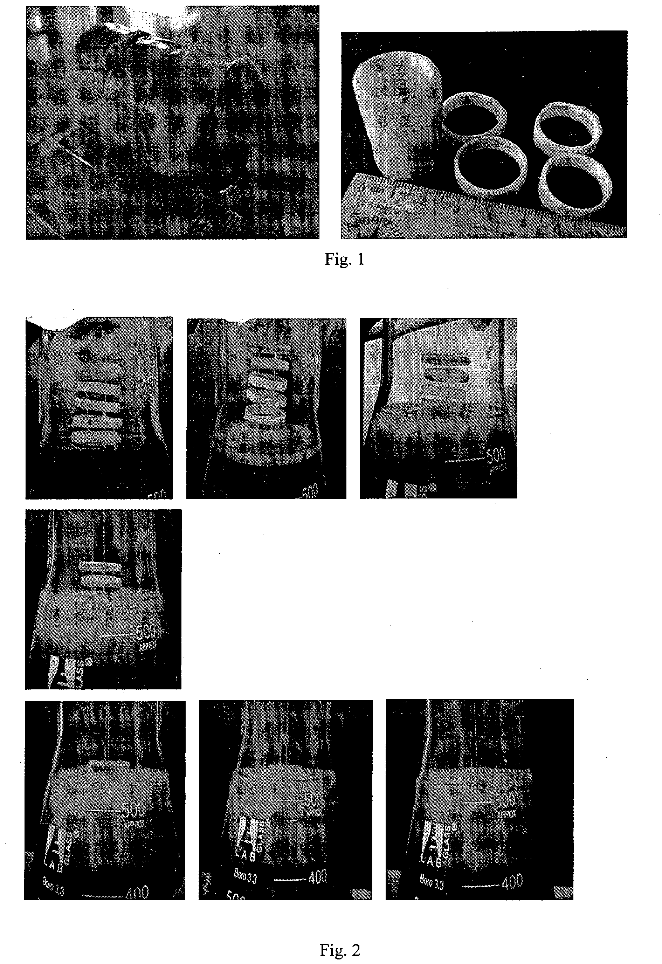

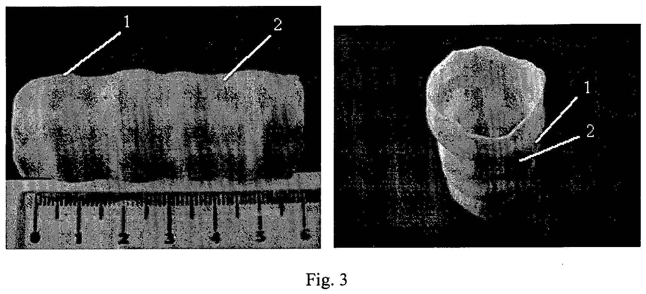

The subject of the invention was shown as an exemplar model at the accompanying figures: Fig. 1 shows a photograph of an sample construct made from mercerised BioNanoCellulose from which rings forming the prosthesis frame are cut out, Fig. 2 - photographs of subsequent stages of their overgrowing made from mercerised BioNanoCellulose with native BioNanoCellulose and Fig. 3 - photographs of a finished tracheal prosthesis comprising rings made from mercerised BioNanoCellulose having the characteristics of cartilage overgrown with mercerised BioNanoCellulose membrane.

### Example.

A tracheal prosthesis was constructed in the shape of a section of tubular pipe having its outer diameter of 4.5 cm and length of 11 cm and its inner diameter equal to the diameter of the human trachea interior. The framework of its walls was made by rings (1) made from mercerised BioNanoCellulose obtained under the bacterial culture of *Gluconacetobacter xylinus,* having the characteristics of cartilage, with a height of 7 mm, located at a distance of 7 mm from one another, and these rings were overgrown with the membrane (2) made from native BioNanoCellulose obtained under the bacterial culture of *Gluconacetobacter xylinus.*
This prosthesis is made in the following manner: The inoculated substrate with its composition of the following weight parts: 20 weight parts of glucose, 5 weight parts of yeast extract, 5 weight parts of peptone, 2.5 weight parts of MgSO₄x7H₂O, 2.7 weight parts of Na₂HPO₄, 1.15 weight parts of citric acid, 10 weight parts of ethanol and distilled water up to 1000 parts was inoculated with 5% (v/v) suspension of *Gluconacetobacter xylinum* bacteria (5x107 cfu/ml) kept in the substrate for 7 days at 4°C and this inoculum was grown for 2 days at a temperature of 30°C. And this cultivated inoculum of bacteria was applied to inoculate the production substrate with the same composition using: 5% suspension (v/v) and 10 weight parts of ethanol; then the whole volume was preincubated for 24 hours at a temperature of 30°C and afterwards the inoculated substrate was transferred to a glass flask with its side walls inclined in relation its bottom at an angle of 25° and the diameter of its neck equal to 4.5 cm. The height of this substrate from the bottom of this flask to the place where BioNanoCellulose membrane is formed was equal to 11 cm. The cultivation was conducted under the stationary conditions at a temperature of 30°C for 28 days. Under these conditions the construct made from BioNanoCellulose in the shape of a cylinder with its length of 11 cm and diameter of 4.5 cm was fitted - by means of a concentric blanking die - with a throughway hole with its diameter equal to the diameter of the human trachea interior, and - upon placing a glass tube with its diameter equal to the diameter of the human trachea interior in the drilled construct - the construct was treated with the process of mercerisation using 20% aqueous solution of sodium hydroxide for 24 hours. As a result of this process of mercerisation, a tube was formed out of mercerised BioNanoCellulose having the character istics of cartilage from which rings were cut with their width of 7 mm.
The glass bioreactor with its walls inclined in relation to its bottom at an angle of 75° was fitted with a structure to suspend such rings made of BioNanoCellulose, composed of a rotary roller with an element with hooks wound around intended to suspend them vertically. Upon suspending such rings arranged one above the other at a distance of 7 mm from one another at hooks, they were subjected to the process of thermal sterilisation at a temperature of 121°C for 20 minutes under pressure of 1013 hPa. Upon the process of sterilisation, the bioreactor was filled with the medium with its composition specified above and inoculated with an inoculum of *Gluconacetobacter xylinum* bacteria, then these suspended rings were lowered so that the surface of the lowest one touches the medium surface and the culture was cultivated at a temperature of 30°C for 18 days, during which - in the course of growth of native BioNanoCellulose layers at the medium surface - subsequent rings were lowered into the medium. Upon all the rings have overgrown with BioNanoCellulose, the remaining composite parts were released from the suspension and taken out of the bioreactor. The resulting composite was fitted - by means of a concentric blanking die - with a concentric throughway hole with its diameter smaller by 3 mm from the one of the rings grown into BioNanoCellulose and the resulting composite was successively rinsed in hot tap water, treated with 1% aqueous solution of sodium hydroxide at a temperature of 100°C for 1 hour, rinsed in tap water, treated with 1% aqueous solution of acetic acid for 24 hours, re-rinsed in tap water, and finally rinsed in distilled water until the rinsing solution reached pH 7; then excess water was taken off the composite in order to obtain its wall 3 mm in thickness.

In this manner it led to the production of composite with its walls made of native BioNanoCellulose strengthened with rings made of mercerised BioNanoCellulose used to prevent the narrowing of such a prosthesis when bended and twisted. The resulting composite had the appearance and dimensions of a biological section of the human trachea.

## Claims

1. Tracheal prosthesis made from BioNanoCellulose obtained under the production culture of *Gluconacetobacter xylinus* bacteria, in the form of a tube-shaped segment with its inner diameter equal to the diameter of the human trachea interior is **characterised by that** its walls have rings (1) made from mercerised BioNanoCellulose obtained under the culture of *Gluconacetobacter xylinus* bacteria, in the form of cartilage, with its height of 7-10 mm, arranged at a distance of 4-7 mm from one another, forming the prosthesis frame, which is overgrown with native BioNanoCellulose membrane (2) obtained under the culture of *Gluconacetobacter xylinus* bacteria.

2. Method of construction of the tracheal prosthesis specified in the Claim 1, from BioNanoCellulose under the stationary production culture of a strain *of Gluconacetobacter xylinus* bacteria at the production substrate containing 20 weight parts of glucose, 5 weight parts of yeast extract, 5 weight parts of peptone, 2.5 weight parts of MgSO₄ x 7 H₂O, 2.7 weight parts of Na₂HPO₄, 1.15 weight parts of citric acid, 10 weight parts of ethanol, up to 1000 weight parts of distilled water, inoculated with an inoculum of the bacteria kept at the inoculated substrate having the same composition, at a temperature of 30°C within 3-28 days and then mercerised in an aqueous solution of sodium hydroxide, under the process of overgrowth of the material with native BioNanoCellulose membrane suspended vertically in the bioreactor with walls inclined in relation to the base, having the production culture substrate of a strain of *Gluconacetobacter xylinus* bacteria at its raised composition and lowered gradually into the substrate on subsequent days of cultivation of the bacteria under the conditions specified above, in the course of growth of native BioNanoCellulose layers at the medium surface until overgrowth of the suspended material with BioNanoCellulose membrane over the entire surface, is **characterised by that** at first under such bacterial culture a construct is made from native BioNanoCellulose in the shape of a cylinder with its diameter of 3-8 cm and length of 8-30 cm in which a concentric, throughway hole having its diameter equal to the diameter of the human trachea interior and then is subjected to the process of mercerisation, and then, using the prosthesis frame made in this manner from mercerised BioNanoCellulose, having the characteristics of cartilage, rings with their width of 7-10 mm are cut out, and then they are - connected or not connected, arranged one above the other at a distance from each other equal to 4-7 mm, after their sterilisation - subjected to the process of overgrowing with native BioNanoCellulose through lowering subsequent rings into the medium placed in the bioreactor with its walls inclined in relation to its base, in the subsequent days of the cultivation, and then - when overgrown with native BioNanoCellulose - these rings are taken out from the bioreactor, a concentric throughway hole with its diameter smaller by 2-3 mm from the diameter of the rings grown into BioNanoCellulose and the prosthesis made in the manner is treated with the process of purification.

3. The method according to the Claim 2 is **characterised by that** the construct from native BioNanoCellulose in the shape of a cylinder is obtained under the production culture of bacteria in a glass flask with its side walls inclined in relation to its bottom at an angle of 10-80°, with the diameter of its neck equal to 3-8 cm and the height of the medium layer from the flask bottom to the place where BioNanoCellulose is formed equal to 8-30 cm.

4. The method according to the Claim 2 is **characterised by that** the construct made from native BioNanoCellulose is treated with the process of mercerisation upon placing a glass tube with the same diameter as the one of the the trachea interior into a hole drilled in it while the process of mercerisation is run using 20-30% aqueous solution of sodium hydroxide for 24 hours at room temperatures.

5. The method according to the Claim 2 is **characterised by that** the rings cut from the construct made from native BioNanoCellulose are treated - prior to their overgrowth with native BioNanoCellulose - with thermal sterilisation at a temperature of 121°C for 20 minutes under pressure of 1013 hPa.

6. The method according to the Claim 2 is **characterised by that** the rings cut out of the construct made from native BioNanoCellulose are subjected to their overgrowth with native BioNanoCellulose in the bioreactor with its walls inclined at an angle of 10-80° in relation to the base, while - prior to starting the culture - they are lowered into the reactor until the lowest ring gets in contact with the medium surface.

7. The method according to the Claim 2 is **characterised by that** the process of purification of the resulting prosthesis consists first in rinsing it with hot tap water, treating with 1% aqueous solution of sodium hydroxide at a temperature of 100°C for 1 hour or 1-2% aqueous solution of this hydroxide for 20-24 hours at room temperatures, rinsing in tap water, treating with 1% aqueous solution of acetic acid for 20-24 hours, re-rinsing in tap water, rinsing in distilled water and finally removing excess water off the composite in order to obtain its wall 2-3 mm in thickness, afterwards the prosthesis is packaged and sterilised.

## Patentansprüche

1. Tracheale Bionanocellulose-Prothese, erhalten durch die Produktion von stationären *Gluconacetobacter xylinus*-Bakterien in Form eines Röhrenabschnitts mit einem Innendurchmesser, der gleich dem Durchmesser der Luftröhre ist, **dadurch gekennzeichnet, dass** ihre Wände Ringe (1) aus merzerisierter Bionanozellulose enthalten, erhalten in der *Gluconacetobacter xylinus-Kultur,* Knorpel, 7 - 10 mm hoch, im Abstand von 4 - 7 mm, bildet das Rückgrat der Prothese, die mit der Membran (2) aus nativer Bionanozellulose, die durch Kultivieren von *Gluconacetobacter xylinus* erhalten wurde, überwachsen ist.

2. Verfahren zur Herstellung einer wie in Anspruch 1 definierten trachealen Prothese aus Bionanozellulose, erhalten auf dem Wege der Produktionszucht eines Bakterienstammes aus Gluconacetobacter xylinus, auf einer Herstellungsbasis die 20 Gewichtsteile Glucose, 5 Gewichtsteile Hefeextrakt, 5 Gewichtsteile Pepton, 2,5 Gewichtsteile MgSO 4 x 7H 2 O, 2,7 Gewichtsteile Na 2 HPO 4, 1,15 Gewichtsteile Zitronensäure, 10 Gewichtsteile Ethanol, bis zu 1000 Gewichtsteile destilliertes Wasser und angeimpftes Inokulum dieser Bakterien enthält, gelagert auf einem Inokulum mit der gleichen Zusammensetzung bei einer Temperatur von 30°C für 3 - 28 Tage, dann merzerisiert in einer wässrigen Lösung von Natriumhydroxid unter Verwendung des Überwucherungsprozesses mit einer Membran aus Bionanozellulose aus nativem Material, vertikal in einem Bioreaktor mit zu ihrer Basis geneigten Wänden aufgehängt und ein Produktionskulturmedium enthaltend, einen Gluconacetobacter xylinus-Stamm mit der obigen Zusammensetzung, langsam in den Boden des Mediums abgesenkt zwecks Durchführung der Zucht von Bakterienkulturen unter den oben angegebenen Bedingungen, mit Zunahme dernativen Schichten aus Bionanocellulose auf der Oberfläche des Mediums bis zur Suspension des suspendierten Materials mit einer Bionanozellulose-Membran über die gesamte Oberfläche, **dadurch gekennzeichnet, dass** zuerst auf dem Wege der Bakterienzüchtung ein Konstrukt aus nativer Bionanozellulose hergestellt wird, zylindrisch in der Form, mit 3 - 8 cm Durchmesser und 8 - 30 cm Länge, in dem wiederum ein konzentrisches Durchgangsloch hergestellt wird mit einem Durchmesser, der dem Durchmesser des Inneren der menschlichen Luftröhre gleicht, woraufhin aus dem so hergestellten Skelett eine merzerisierte Bionanozellulose-Prothese erstellt wird, mit Knorpelcharakter werden Ringe von 7 - 10 mm Breite ausgeschnitten, die verbunden oder nicht verbunden - übereinander in 4 - 7 mm Abstand angelegt sind und nach ihrer Sterilisierung der Verwachsung mit nativer Bionanozellulose unterzogen werden, indem die Ringe aufeinanderfolgend in den Bioreaktor mit geneigten Wänden relativ zu ihrer Basis abgelassen werden, in den nachfolgenden Tagen der Kultivierung der Bakterien unterzogen, woraufhin die mit Bionanozellulose überwachsenen nativen Ringe aus dem Bioreaktor herausgenommen werden, dann wird ein konzentrisches Durchgangsloch mit einem Durchmesser geschnitten, um 2-3 mm kleiner als der Durchmesser der in der so hergestellten Bionanozellulose eingebetteten Ringe, woraufhin die so hergestellte Prothese einem Reinigungsprozess unterzogen wird.

3. Verfahren nach Anspruch 2, **dadurch gekennzeichnet, dass** das zylindrische Bionanozellulose-Konstrukt durch Kultivieren des Bakteriums in einem Glaskolben mit gegenüber dem Boden geneigten Seitenwänden in einem Winkel von 10 - 80 ° erhalten wird, wobei der Durchmesser des Halses gleich 3 - 8 cm beträgt ist, die Höhe der mittleren Schicht vom Boden des Kolbens bis zum Ort der Bildung des Bionanozellulose-Films beträgt 8 bis 30 cm.

4. Verfahren nach Anspruch 2, **dadurch gekennzeichnet, dass** das native Bionanozellulose-Konstrukt einer Merzerisierung unterzogen wird, nachdem es in die Bohrung einer Glasröhre mit einem Durchmesser gleich dem Innendurchmesser der Trachea platziert worden ist, wobei die Merzerisierung mit einer 20- bis 30-prozentigen wässrigen Natriumhydroxidlösung innerhalb von 24 Stunden durchgeführt wird.

5. Verfahren nach Anspruch 2, **dadurch gekennzeichnet, dass** die aus dem nativen Bionanzellulose-Konstrukt geschnittenen Ringe vor dem Überwachsen mit nativer Bionanozellulose einer thermischen Sterilisation bei 121 ° C für 20 Minuten unter einem Druck von 1013 hPa unterzogen werden.

6. Verfahren nach Anspruch 2, **dadurch gekennzeichnet, dass** die aus dem nativen Bionanozellulose-Konstrukt ausgeschnittenen Ringe mit nativer Bionanzellulose in einem Bioreaktor mit Wänden, die in einem Winkel von 10 bis 80 ° zu ihrer Basis geneigt sind, verschmutzt werden, bevor sie in den Reaktor abgegeben werden bis der untere Ring die Oberfläche des Mediums berührt.

7. Verfahren nach Anspruch 2, **dadurch gekennzeichnet, dass** das Verfahren zur Reinigung der hergestellten Prothese in aufeinanderfolgender Spülung in heißem Leitungswasser, Behandlung mit einprozentiger wässriger Natriumhydroxidlösung bei 100 ° C für 1 Stunde oder mit ein- bis zweiprozentiger wässriger Lösung dieses Hydroxids über einen Zeitraum von 20 - 24 Stunden bei Raumtemperatur, Spülen in Leitungswasser, Behandlung mit einprozentiger wässriger Essigsäure für einen Zeitraum von 20 - 24 Stunden, erneutes Spülen in Leitungswasser, Spülen in destilliertem Wasser und abschließendes Komprimieren des Kompositums mit überschüssigem Wasser bis zu einer Wandstärke von 2 - 3 mm, woraufhin die Prothese verpackt und sterilisiert wird.

## Revendications

1. La prothèse trachéale de bionanocellulose obtenue par la production de culture stationnaire de bactérie *Gluconacetobacterxylinus,* en forme de section de tube de diamètre intérieur égal au diamètre de la trachée, **caractérisée en ce que** ses parois contiennent des anneaux (1) de bionanocellulose mercerisée obtenue par la culture de bactérie *Gluconacetobacterxylinus,* à caractère du cartilage, de 7-10 mm de haut, situés à 4-7 mm de distance, constituant le squelette de la prothèse, qui est couvert par la membrane (2) de la bionanocellulose native obtenue par la culture de bactérie *Gluconacetobacterxylinus.*

2. Le procédé de préparation de la prothèse trachéale de bionanocelulose, décrite dans la revendication 1, obtenue par la production de culture stationnaire de la souche bactérienne *Gluconacetobacterxylinus* sur le milieu de production comprenant : 20 parties en poids de glucose, 5 parties en poids d'extrait de levure, 5 parties en poids de peptone, 2,5 parties en poids de MgSO4 x 7 H₂O, 2,7 parties en poids de Na₂HPO₄, 1,15 parties en poids d'acide citrique, 10 parties en poids d'éthanol, jusqu'à 1000 parties en poids d'eau distillée, inoculé avec l'inoculum de ces bactéries, conservé sur le milieu d'inoculum de la même composition à 30° C pendant 3-28 jours, puis mercerisée dans une solution aqueuse d'hydroxyde de sodium, en utilisant le procédé de recouvrement par la membrane de bionanocelulose native de matériau suspendu verticalement dans un bioréacteur avec des parois inclinées par rapport à sa base, contenant le milieu de culture de production de la souche bactérienne *Gluconacetobacterxylinus* avec la composition décrite ci-dessus et trempé progressivement dans le milieu au cours des jours de la culture des bactéries, menée dans les conditions indiquées ci-dessus, avec des couches de bionanocelulose native de plus en plus épaisses à la surface du support jusqu'à ce que le matériau suspendu soit couvert sur toute la surface, **caractérisé en ce que** dans un premier temps, grâce à la culture de bactéries, une construction de bionanocellulose native est préparée, en forme de cylindre d'un diamètre de 3-8 cm et d'une longueur de 8-30 cm, qui possède un trou concentrique, traversant, d'un diamètre égal au diamètre de l'intérieur de la trachée humaine, et ensuite cette construction est mercerisée, après quoi, à partir du squelette ainsi obtenu de la prothèse de bionanocellulose mercerisée, à caractère de cartilage, sont découpés les anneaux d'une largeur de 7-10 mm, assemblés ou non, situés l'un sur l'autre à une distance de 4-7 mm l'un de l'autre, qui sont, après leur stérilisation, soumis au recovrement de bionanocellulose native lors de trempage des anneaux subséquents dans le milieu placé dans un bioréacteur avec des parois inclinées par rapport à sa base, au cours des jours de la culture bactérienne, après quoi les anneaux couverts par la bionanocellulose native sont sortis du bioréacteur, et un trou traversant, concentrique, de diamètre inférieur de 2 à 3 mm par rapport au diamètre des anneaux couverts par la bionanocellulose, est découpé dans cette construction, après quoi la prothèse, créée de cette façon est ensuite nettoyée.

3. Le procédé selon la revendication 2, **caractérisé en ce que** la construction en bionanocellulose native, en forme d'un cylindre est obtenue en cultivant les bactéries dans une fiole en verre avec les parois latérales inclinées par rapport au fond sous l'angle de 10-80° et qui a le diamètre du col égal à 3-8 cm, et la hauteur de la couche du milieu à partir du fond de la fiole jusqu'à l'endroit de la formation du film de bionanocellulose égale à 8-30 cm.

4. Le procédé selon la revendication 2, **caractérisé en ce que** la construction de bionanocellulose native est soumise à mercerisation après avoir placée dans un trou creusé dans cette construction d'un tube de verre de diamètre égal au diamètre intérieur de la trachée, la mercerisation étant réalisée avec une solution aqueuse à 20-30% d'hydroxyde de sodium pendant 24 heures à température ambiante.

5. Le procédé selon la revendication 2, **caractérisé en ce que** les anneaux découpés dans la construction
de bionanocellulose native, sont stérilisés thermiquement à 121° C pendant 20 minutes à 1013 hPa, avant qu'ils soient couverts par la bionanocellulose native.

6. Le procédé selon la revendication 2, **caractérisé en ce que** les anneaux découpés dans la construction
de bionanocellulose native, sont soumis au recouvrement par la bionancellulose native dans un bioréacteur dont les parois sont inclinées de 10 à 80° par rapport à sa base, mais avant de commencer la culture, les anneaux sont mis dans le réacteur de façon que l'anneau inférieure entre en contact avec la surface du milieu.

7. Le procédé selon la revendication 2, **caractérisé en ce que** le processus de nettoyage de la prothèse réalisée consiste en un rinçage successif à l'eau chaude du robinet, un traitement avec une solution aqueuse à 1% d'hydroxyde de sodium à 100° C pendant 1 heure ou avec une solution aqueuse à 1-2% de cet hydroxyde pendant 20-24 heures à température ambiante, un rinçage à l'eau du robinet, un traitement avec de l'acide acétique aqueux à 1% pendant 20-24 heures, un autre rinçage à l'eau du robinet, un rinçage à l'eau distillée et une compression finale du composite afin d'enlever l'excès de l'eau jusqu'à ce que ses parois aient 2-3 mm d'épaisseur, après quoi la prothèse est emballée est stérilisée.
